# EUROPEAN PATENT APPLICATION

(11) **EP 1 182 265 A1**
(43) Date of publication of application: **27.02.2002**
(21) Application number: 00810726.0
(22) Date of filing: 15.08.2000
(51) Int. Cl.: C12Q 1/68

(54) **Method for determining genetic traits of improved breed animal embryos prior to implantation**

(71) Applicant: Eidgenössische Technische Hochschule Zürich, 8092 Zürich (CH)
(72) Inventor: Jörg, Hannes, Dr., 3413 Kaltacker (CH)
(74) Representative: Liebetanz, Michael, Dipl.-Phys.

(57) **Abstract**

The present invention relates to a new method for determining genetic traits of improved breed animal embryos prior to their implantation into a female carrier. More particularly the invention provides a new process for predicting the sex by detecting single nucleotide polymorphisms and microsatellite markers on the X chromosome. Furthermore, the invention provides the opportunity to analyse simultaneously the sex, the parentage and other genetic traits of an embryo.

## Description

The present invention relates to a new method for determining genetic traits of improved breed animal embryos prior to their implantation into a female carrier. The determination is based on a detection of single nucleotide polymorphism and microsatellite markers. Furthermore, the invention relates to laboratory kits for carrying out said determination.

The ability to determine genetic traits in animals prior to implantation would provide numerous advantages in the livestock and dairy industries as well as in veterinary medicine. In the dairy and beef cattle industry advances in embryo research have resulted in a great demand for a method of quickly determining for example the sex, controlling parentage or typing of various genetic traits by taking cells from an embryo at early stages of development. The commercial efficiency of livestock and dairy operations would be greatly improved by allowing gestation to be established with embryos having the desired genetic traits.

In situations where for economic or health reasons a determination of the sex, parentage or specific genetic traits of an embryo is indicated, it is important to carry out the respective determination as soon as possible after fertilisation. There is a substantial increase in risk to the life and health of a female if abortion is induced after gestation. Furthermore, with livestock an abortion is commercially very inefficient because of reduced reproductive efficiency. In the livestock and dairy industry, where embryo transfer is extensively practised, a determination of the desired genetic traits even before transfer of the fertilised cells would be the most preferred way of handling. All risks and costs associated with pregnancy and abortion could be avoided.

To date, genotyping of animal embryos has been used for sexing. Several methods can be used for sexing embryos, including: karyotyping, H-Y antigen analysis, X-chromosome-linked enzymatic activity test, DNA hybridisation and PCR methods.

The karyotyping method is relied principally on cytogenetic analysis against blastomeres of an embryo and determines the sex type of the embryo on the basis of the occurring of sex chromosomes or the observation of Barr's body. The detection of a Barr's body is not a suitable method for sexing embryos of common domestic animals, since, in somatic cells of these animals, Barr's body is observed in only less than 50%. Furthermore, in the cytoplasm of embryos of common domestic animals including embryos of pigs, cattle, sheep and goat, an observation of Barr's body is difficult to carry out, because the X inactivation, which leads to the Barr's body, starts during the blastula stage and the embryo transfer.

At present, karyotyping done on embryos of domestic animals consists usually of treating the embryo firstly with colchicine to induce the entry of its blastomere into metaphase of cell division and verifying whether there is a Y chromosome in the karyotype of the embryo according to the shape of the chromosome. However, by sexing embryos based on karyotyping the identifiability of a cell is low, and in particular, with single blastomeres, the probability to obtain an identifiable karyotype is even smaller.

The X-linked enzymatic activity test is based on the fact that both X chromosomes of normal female mammals have certain functions before entry into the blastula stage during the development of the embryo. After the blastula stage one of the two X chromosomes will be inactivated. The activities of several enzymatic genes on the X chromosome (e.g. glucose-6-phosphate dehydrogenase (G6PD) and hypoxanthine phosphoribosyl transferase (HPRT)) can be used to predict the number of X chromosomes present in an embryo. During application of such an enzymatic activity test, the survival rate of the embryo is low due to the direct exposure of the embryo to a reaction solution. Moreover, the results are not always unambiguous and therefore difficult to interpret.

Sex-determination of embryos by detection of male-specific, Y-chromosomal DNA (YCD assay) is based on two molecular biological principles: the first one comprises the use of a Y-specific DNA sequence as a probe for the hybridisation of genomic DNA extracts derived from the embryo to be tested; the second principle comprises the use of specifically designed primers based on Y-specific nucleotide sequences. *In vitro* amplification of Y specific gene sequences by means of PCR is carried out. Because of their high sensitivity and high efficiency, PCR amplifications of Y-chromosome specific genes (e.g. bovine and porcine SRY gene) is the most practical and therefore most commonly applied technique. Autosomal sequences are amplified simultaneously, serving as an internal control to monitor successful biopsies and at the same time the PCR-reaction. PCR reactions with primers homologous to both X and Y chromosomes, which can amplify gene fragments of different lengths simultaneously from the X and Y chromosome, such as bovine amelogenin genes, are commercially available.

YCD assays provide quick results. However, the presence or absence of amplified fragments from Y-chromosome specific gene sequences can be due to reasons other than the sex of the embryo to be tested. PCR amplification is extremely sensitive and can fail for many reasons. False negative or false positive results may occur. False positive results are often due to unspecific amplification and/or contamination, mainly from sperm.

It is the object of the present invention to overcome the above mentioned disadvantages and to provide a new method which is able to deliver an absolutely unambiguous result as far as the sexing of improved breed embryos is concerned.

Furthermore, the invention described here provides a method for analysing the genotypes of almost all desired genetic traits, like for example red factor, polledness, double muscling, malignant hyperthermina, oedema, Weaver, BLAD, DUMPS, Syndactyly, estrogen receptor.

Another object of the present invention is the possibility of carrying out a parentage control when mixed semen is used or simultaneous fertilisation of several oocytes is applied in order to achieve a successful *in vitro* fertilisation.

Still another objcect of the present invention is the provision of laboratory kits for carrying out the respective tests.

A further, important aspect of the present invention is, that the novel method described here for determination of genetic traits, such as the determination of sex or coat colour, is carried out prior to the transfer of the embryo into the female.

The present invention provides a far better accuracy for sexing an embryo than the methods described in the state of the art, because the results do not depend on the presence or absence of PCR products. Additionally, the system is able to detect contamination.

The present invention accomplishes this by providing a new method which is based on a characterisation and identification of polymorphic microsatellite markers on the X-chromosome. This system provides a far better accuracy than Y chromosome detection systems because the results depend on the allele size and not only on the amplification. The bovine genetic map contains more than 60 X chromosome specific microsatellite markers which can be used in many combinations to have different alleles between father and mother.

The invention is illustrated by the following figures wherein
Figur 1 shows sexing of embryos;
Figur 2 shows parentage control, and
Figur 3 shows typing of genetic traits and sexing in combination

The multiplex PCR provides the opportunity to further analyse each known amplified fragment polymorphism, e.g. single nucleotide polymorphism (SNP) or variable numbers of tandem repeats (VNTRS). If a restriction fragment length polymorphism is used to detect a SNP in combination with several VNTRs, the specific fragment will be amplified in a second PCR. The PCR product will be restriction enzyme digested with the specific enzyme and analysed separately or together with the VNTRs.

For sexing of embryos a polymorphic microsatellite marker from chromosome X is amplified and a fragment length analysis is done. A male embryo has only one allele which is derived from the mother. A female embryo has two alleles, one is the same as that of the father (Figure 1). Sperm contamination can be detected by a simultaneous analysis of a marker from an autosome which will have both alleles from the father. The method for determining the sex of an improved breed embryo comprises the following steps:
a) isolation of an embryo cell or cells at the blastula stage by microsection or biopsy;
b) denaturating DNA from said cells by alternately freezing and thawing the cells;
c) performing of polymerase chain reaction (PCR) amplification on said denatured DNA to amplify a fragment homologous to a region of the X chromosome comprising microsatellite markers; and
d) evaluating the size of the fragments obtained from step c or evaluating their sequence.

Typing of genetic traits for example, could comprise the selection of female red embryos by crossing two black carriers of the recessive red allele. This results in six different genotypes (red/female; heterozygote/female; black/female; red/male; heterozygote/male; black/male). From such a cross, 12.5 % of the embryos are expected to be red and female. An amplified RFLP assay provides the fragments which are associated with the desired genotype (Figure 3).

The method for determining the sex and the coat colour of an improved breed embryo comprises the following steps:
a) isolation of an embryo cell or cells at the blastula stage by microsection or biopsy;
b) denaturating DNA from said cells by alternately freezing and thawing the cells;
c) performing a first round of polymerase chain reaction (PCR) on said denatured DNA to amplify a fragment homologous to a region of the X chromosome comprising microsatellite markers and single nucleotide polymorphisms using primers designed from the bovine melanocyte stimulating hormone receptor GenBank accession number BT39469
   (http://www.ncbi.nlm.nih.gov:80/entrez/query.fcgi?cmd=Retrieve&db=Nucleotide&list_uids=6091552&dopt=GenBank);
d) performing a second round of PCR on a DNA aliquot from the first PCR round using primers of the microsatellite markers in one reaction and two primers of the melanocyte stimulating hormone receptor in another reaction.
e) performing a restriction enzyme digest with the DNA derived from PCR with the two primers of the melanocyte stimulating hormone receptor from step d); and
f) evaluating the size of the fragments obtained from step d and step e.
Instead of performing steps e and f the fragment derived from step d can be evaluated by its sequence, for example by microsequencing, hybridisation methods, ligation, etc.

Parentage control is necessary when mixed semen is used to obtain more embryos or simultaneous fertilisation of several oocytes is applied for successful in vitro fertilisation which can lead to embryos of an unknown father and mother. A parentage control of the embryo with 12 unlinked microsatellite markers with an exclusion probability of more than 99% will determine the origin of the embryo (Figure 2). The method for controlling parentage of an improved breed embryo comprises the following steps:
a) isolation of an embryo cell or cells at the blastula stage by microsection or biopsy;
b) denaturating DNA from said cells by alternately freezing and thawing the cells;
c) performing of polymerase chain reaction (PCR) amplification on said denatured DNA to amplify fragments of different chromosomes comprising microsatellite markers; and
d) evaluating the size of the fragments obtained from step c or by evaluating the sequence of the fragments obtained in step c.

### EXAMPLE 1

### Biopsy of day 7 blastocysts

After transfering the embryos from culture medium into ViGro Holding Plus medium (AB Technology, Pullman, WA, USA), the cells can be taken either by microsection or by biopsy. Microsection was done by cutting through the zona pellucida and slicing off a few cells using a splitting blade. The biopsies were taken with a pair of mikromanipulators under an inverted microscope (Axiovert 100, Carl Zeiss, Germany). Each embryo was fixed with a flame polished holding pipette held in one micromanipulator. A bevelled biopsy pipette (diameter 30-50 µm) was stabilised in the electronically controlled micromanipulator. The biopsy pipette was pushed through the zona and a single blastomere was removed.

### EXAMPLE 2

### DNA preparation

In order to analyse every embryonic cell, the blastomeres are placed directly on the bottom of a 0.5 ml PCR tube. Sterile deionized water was added up to 10 µl. The samples were frozen in a -70 °C refrigerator for at least 10 min. The tubes are placed in a thermal cycler (Hybaid Teddington, UK) and heated at a temperature of 95 °C for 5 min. to carry out the pre-treatment of protein denaturation and then immediately cooled to 4 °C or placed on ice.

The microsatellite markers were selected from the 'cattle genome map' of MARC, USDA and the primer sequences of MSHR are designed from the bovine melanocyte stimulating hormone receptor GenBank accession number BT39469. Two multiplex systems were used:

| Multiplex system for parentage control: | | | |
|---|---|---|---|
| Marker | Chromosome | Label | Length range |
| BM1824 | 1 | Joe | 178-192 |
| BM2113 | 2 | Joe | 123-143 |
| BM6465 | 3 | Fam | 108-124 |
| BMS779 | 4 | Fam | 181-193 |
| AGLA293 | 5 | Fam | 230-257 |
| BMS522 | 7 | Joe | 146-154 |
| TGLA73 | 9 | Tamra | 116-130 |
| BM875 | 10 | Tamra | 93-111 |
| TGLA227 | 18 | Fam | 76-102 |
| TGLA122 | 21 | Fam | 137-181 |
| BM1818 | 23 | Tamra | 258-272 |
| BMC2228 | 29 | Joe | 171-177 |

| Multiplex system for sex and coat colour control: | | | |
|---|---|---|---|
| BMS631 | X | Tamra | 131-141 |
| BMS1616 | X | Joe | 93-109 |
| BMC6021 | X | Fam | 139-159 |
| MSHR P9 / P12 | 18 | Fam | 203/238 |

PCR was carried out in a reaction volume of 25 µl containing the 10 µl DNA in water, 10 mM Tris-HCl pH 9.0, 50 mM KCl, 1.5 mM MgCl₂, 200 µM of each deoxynucleotide, 0.4 µM of each primer and 1.25 U of Taq DNA polymerase (Pharmacia Biotech, USA) in a thermal cycler (Hybaid Teddington, UK). After initial denaturation for 5 min at 95 °C, the PCR profile consisted of a denaturation step at 95 °C for 30 s, an annealing step at 58 °C for 30 s and an elongation step at 72 °C for 30 s for a total of 40 cycles, followed by a final extension of 7 min at 72 °C. In case of a restriction fragment length polymorphism, another round of PCR with the two primers for the fragment to be restricted was performed. PCR conditions were as described above, with the exceptions that 1 µl of the first PCR was used as template, and the number of cycles was 30. The PCR product obtained was digested with MspI restriction enzyme (Boehringer Mannheim, Germany).

An ABI PRISM™ 377 DNA Sequencer in combination with ABI Genescan® software 3.1 (Applied Biosystems, Perkin-Elmer Corp., CA) was used to evaluate the size of the PCR-fragments. 0.5 µl of PCR product were mixed with 2.5 µl deionized formamide, 0.5 µl loading buffer (50mM EDTA, 50 mg/ml blue dextran) and 0.5 µl GeneScan-250[ROX] size standard used as an internal lane standard. After heating at 95 °C for 5 min. 2.0 µl of each sample was loaded into a separate gel lane of a 4% acrylamide denaturing gel. Depending on the length of the gel used, the GS 12A-2400 or the GS 36A-2400 run module was used. The allele peaks are detected by their typical pattern for dinucleotide repeats with the associated PCR stutter bands within a maximum range of eight base pairs from the allele peak. An individual is hetreozygote, if two allele peaks are visible in the size range of the marker and these peaks differ by more than 2 base pairs.

### EXAMPLE 3

### Potential problems with contamination

### Media used in embryo manipulation

Fetal calf serum (FCS) or bovine serum albumin (BSA) used in embryo holding medium are occasionally contaminated with amplifiable DNA. A contamination shosn by non parental alleles.

### Cells adhering to the zona pellucida

The small size of the biopsy (< 10 µm) can cause a mix-up with other small cells in the microdroplet. The embryo can either be treated to remove all cells adhering to the zona pellucida or the biopsy can be taken under microscopic control.
Taking cumulus cells by mistake instead of or together with an embryo biopsy would result in the presence of all alleles of the mother.
Spermatozoa can be detected by the presence of more than two alleles of autosomal microsatellite markers.

### Environmental contamination

Contamination by carry-over of cells or cellular debris from one embryo to the next, by air-borne bovine material or by previous assays can be eliminated due to exercising care during set-up of the assay. Any contamination will lead to non parental alleles.

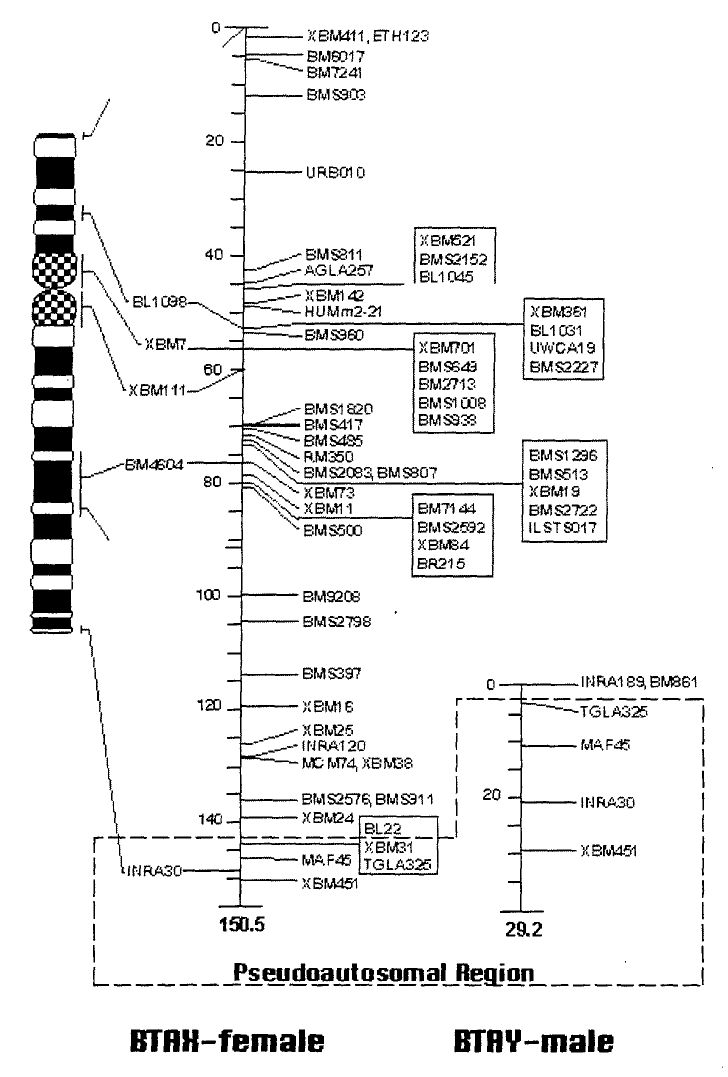
0
XBM411, ETH123
BM6017
BM7241
BMS903
20
URB010
XBM521
BMS2152
BL1045
40
BMS811
AGLA257
XBM142
HUMm2-21
BL1098
XBM361
BL1031
UWCA19
BMS2227
BMS360
XBM7
XBM701
BMS649
BM2713
BMS1008
BMS939
60
XBM111
BMS1820
BMS417
BMS485
RM350
BMS1296
BMS513
XBM19
BMS2722
ILSTS017
BM4604
BMS2083, BMS807
80
XBM73
XBM11
BM7144
BMS2592
XBM84
BR215
BMS500
100
BM9208
BMS2798
BMS397
0
INRA189, BM861
120
XBM16
TGLA325
XBM25
INRA120
MAF45
MCM74, XBM38
20
BMS2576, BMS911
INRA30
140
XBM24
BL22
XBM31
TGLA325
XBM451
MAF45
INRA30
XBM451
150.5
29.2
Pseudoautosomal Region
BTAX-female
BTAY-male

Appendix 1: X-chromosome

Cattle Marker BMS631 (710)
Chromosome: XYLocus: BMS631
Relative Position: 0cM
Region: p24
Database Name: GENBANK Accession Number: G18870

Heterozygosity: 69%; Informative Meioses: 306; Number of Al-
leles: 6
Minimum Allele Size: 131(bp)
Maximum Allele Size: 139 (bp)
Null Alleles: At least one allele identified does not amplify.
Annealing Temperature: 58°C
Forward Primer Sequence: TTTCCACCCTCAGCTAAAGTG
Reverse Primer Sequence: AAATGACAGGTGTCTTGGGC

References:

Title: "A SMALL-INSERT BOVINE GENOMIC LIBRARY HIGHLY ENRICHED
FOR MICROSATELLITE REPEAT SEQUENCES"
Journal: MAMM GENOME, 1995, Volume:6, Page:714
Authors: STONE, RT; PULIDO, JC; DUYK, GM; KAPPES, SM;
KEELE, JW; BEATTIE, CW.

Title: "AN INTEGRATED GENETIC AND PHYSICAL MAP OF THE BOVINE X
CHROMOSOME"
Journal: MAMM GENOME, 1997, Volume:8, Page:16
Authors: SONSTEGARD, TS; LOPEZ-CORRALES, NL; KAPPES, SM; STONE,
RT;
AMBADY, S; PONCEDELEON, FA.

Title: "A SECOND-GENERATION LINKAGE MAP OF THE BOVINE GENOME"
Journal: GENOME RES,1997, Volume:7, Page:235
Authors:  KAPPES, SM; KEELE, JW; STONE, RT; MCGRAW, RA;
SONSTEGARD, TS; SMITH, TPL; LOPEZ-CORRALES, NL; BEATTIE, CW.


Cattle Marker **BMS1616** (1075)

Chromosome: XYLocus: BMS1616
Relative Position: 45.9cM
Region: Not physically assigned
Database Name: GENBANK Accession Number: G18677
Heterozygosity: 72%; Informative Meioses: 247; Number of Al-
leles: 9
Minimum Allele Size: 93(bp)
Maximum Allele Size: 109(bp)
Null Alleles: At least one allele identified does not amplify.
Annealing Temperature: 58°C
Forward Primer Sequence: CAGTGTGTATAGCATGATTCCG
Reverse Primer Sequence: AGTTGGTCTGCATTCATACATTAA

References:

Title: "A SMALL-INSERT BOVINE GENOMIC LIBRARY HIGHLY ENRICHED
FOR MICROSATELLITE REPEAT SEQUENCES"
Journal: MAMM GENOME, 1995, Volume:6, Page:714
Authors: STONE, RT; PULIDO, JC; DUYK, GM; KAPPES, SM;
KEELE, JW; BEATTIE, CW.

Title: "A SECOND-GENERATION LINKAGE MAP OF THE BOVINE GENOME"
Journal: GENOME RES,1997, Volume:7, Page:235
Authors: KAPPES, SM; KEELE, JW; STONE, RT; MCGRAW, RA;
SONSTEGARD, TS; SMITH, TPL; LOPEZ-CORRALES, NL; BEATTIE, CW.

Cattle Marker BMC6021 (729)

Chromosome: XYLocus: BMC6021
Relative Position: 91.3 cM
Region: q27
Database Name: GENBANK Accession Number: G19109
Heterozygosity: 48%; Informative Meioses: 226; Number of Al-
leles: 7
Minimum Allele Size: 139 (bp)
Maximum Allele Size: 159 (bp)
Null Alleles: At least one allele identified does not amplify.
Annealing Temperature: 58°C
Forward Primer Sequence: AGGCCAAGTGAAGAGGTCAC
Reverse Primer Sequence: TCTGCTCCAGCTTTCCAGTA

References:

Title: "DEVELOPMENT OF ABOVINE X CHROMOSOME LINKAGE GROUP AND
PAINTING PROBES TO ASSESS CATTLE, SHEEP, AND GOAT X CHROMOSOME
SEGMENT HOMOLOGIES"
Journal: PROC NATL ACAD SCI USA,1996, Volume:93, Page:3450
Authors: PONCE DE LEON, FA; AMBADY, S; HAWKINS, GA; KAPPES, SM;
BISHOP, MD; ROBL, JM; BEATTIE, CW.

Title: "A SECOND-GENERATION LINKAGE MAP OF THE BOVINE GENOME"
Journal: GENOME RES,1997, Volume:7, Page:235
Authors: KAPPES, SM; KEELE, JW; STONE, RT; MCGRAW, RA;
SONSTEGARD, TS; SMITH, TPL; LOPEZ-CORRALES, NL; BEATTIE, CW.

Appendix 2: Microsatellite markers for parentage control

Cattle Marker TGLA227 (577)

Chromosome: 18 Locus: TGLA227
Relative Position: 84.7 cM
Region: Not physically assigned
Heterozygosity: 86%; Informative Meioses: 345; Number of Al-
leles: 13
Minimum Allele Size: 76 (bp)
Maximum Allele Size: 102 (bp)
Annealing Temperature: 56 °C

Forward Primer Sequence: CGAATTCCAAATCTGTTAATTTGCT
Reverse Primer Sequence: ACAGACAGAAACTCAATGAAAGCA
Ease of Scoring: 1 (1=easy, 5=difficult)

References:

Title: "POLYMORPHIC DNA MARKERS IN BOVIDAE (WORLD INTELLECTUAL
PROPERTY ORG GENEVA)"
Journal: WO PUBL NO 92/13102, 1992, Volume: , Page:
Authors: GEORGES, M ; MASSEY, J ;

Title: "A SECOND-GENERATION LINKAGE MAP OF THE BOVINE GENOME"
Journal: GENOME RES, 1997, Volume: 7, Page: 235
Authors: KAPPES, SM ; KEELE, JW ; STONE, RT ; MCGRAW, RA ;
SONSTEGARD, TS ; SMITH, TPL; LOPEZ-CORRALES, NL ; BEATTIE, CW
;

Cattle Marker BM6465 (337)

Chromosome: 03 Locus: BM6465
Relative Position: 69 cM
Region: Not physically assigned
Database Name: GENBANK Accession Number: G18547
Heterozygosity: 59%; Informative Meioses: 188; Number of Al-
leles: 6
Minimum Allele Size: 108 (bp)
Maximum Allele Size: 124 (bp)
Annealing Temperature: 56 °C
Forward Primer Sequence: AGGAGCAAGCATCTTTAATGTG
Reverse Primer Sequence: TGCCAGGCTATAGAAGGACTT

References:

Title: "A SMALL-INSERT BOVINE GENOMIC LIBRARY HIGHLY ENRICHED
FOR MICROSATELLITE REPEAT SEQUENCES"
Journal: MAMM GENOME, 1995, Volume: 6, Page: 714
Authors: STONE, RT ; PULIDO, JC ; DUYK, GM ; KAPPES, SM ;
KEELE, JW ; BEATTIE, CW ;

Title: "A SECOND-GENERATION LINKAGE MAP OF THE BOVINE GENOME"
Journal: GENOME RES, 1997, Volume: 7, Page: 235
Authors: KAPPES, SM ; KEELE, JW ; STONE, RT ; MCGRAW, RA ;
SONSTEGARD, TS ; SMITH, TPL; LOPEZ-CORRALES, NL ; BEATTIE, CW
;

Cattle Marker TGLA122 (574)

Chromosome: 21 Locus: TGLA122
Relative Position: 67.3 cM
Region: Not physically assigned
Heterozygosity: 72%; Informative Meioses: 320; Number of Al-
leles: 18

Minimum Allele Size: 137 (bp)
Maximum Allele Size: 181 (bp)
Annealing Temperature: 58 °C
Forward Primer Sequence: CCCTCCTCCAGGTAAATCAGC
Reverse Primer Sequence: AATCACATGGCAAATAAGTACATAC

References:

Title: "POLYMORPHIC DNA MARKERS IN BOVIDAE (WORLD INTELLECTUAL
PROPERTY ORG GENEVA)"
Journal: WO PUBL NO 92/13102, 1992, Volume: , Page:
Authors: GEORGES, M ; MASSEY, J ;

Title: "A SECOND-GENERATION LINKAGE MAP OF THE BOVINE GENOME"
Journal: GENOME RES, 1997, Volume: 7, Page: 235
Authors: KAPPES, SM ; KEELE, JW ; STONE, RT ; MCGRAW, RA ;
SONSTEGARD, TS ; SMITH, TPL; LOPEZ-CORRALES, NL ; BEATTIE, CW
;

Cattle Marker BMS779 (688)

Chromosome: 04 Locus: BMS779
Relative Position: 63.9 cM
Region: Not physically assigned
Database Name: GENBANK Accession Number: G18853
Heterozygosity: 41%; Informative Meioses: 137; Number of Al-
leles: 7
Minimum Allele Size: 181 (bp)
Maximum Allele Size: 193 (bp)
Annealing Temperature: 58 °C
Forward Primer Sequence: TCTTGTGGGGAACCTCTA
Reverse Primer Sequence: TGCTTGGAAGTATACATGATGG

References:

Title: "A SMALL-INSERT BOVINE GENOMIC LIBRARY HIGHLY ENRICHED
FOR MICROSATELLITE REPEAT SEQUENCES"
Journal: MAMM GENOME, 1995, Volume: 6, Page: 714
Authors: STONE, RT ; PULIDO, JC ; DUYK, GM ; KAPPES, SM ;
KEELE, JW ; BEATTIE, CW ;

Title: "A SECOND-GENERATION LINKAGE MAP OF THE BOVINE GENOME"
Journal: GENOME RES, 1997, Volume: 7, Page: 235
Authors: KAPPES, SM ; KEELE, JW ; STONE, RT ; MCGRAW, RA ;
SONSTEGARD, TS ; SMITH, TPL; LOPEZ-CORRALES, NL ; BEATTIE, CW
;

Cattle Marker AGLA293 (266)

Chromosome: 05 Locus: AGLA293
Relative Position: 32 cM
Region: Not physically assigned

Heterozygosity: 62%;Informative Meioses: 220; Number of Alleles:
11
Minimum Allele Size: 237 (bp)
Maximum Allele Size: 257 (bp)
Annealing Temperature: 54 °C
Forward Primer Sequence: GAAACTCAACCCAAGACAACTCAAG
Reverse Primer Sequence: ATGACTTTATTCTCCACCTAGCAGA

References:

Title: "POLYMORPHIC DNA MARKERS IN BOVIDAE (WORLD INTELLECTUAL
PROPERTY ORG GENEVA)"
Journal: WO PUBL NO 92/13102, 1992, Volume: , Page:
Authors: GEORGES, M ; MASSEY, J ;

Title: "AN AUTOSOMAL GENETIC LINKAGE MAP OF THE SHEEP GENOME"
Journal: GENETICS, 1995, Volume: 140, Page: 703
Authors: CRAWFORD, AM ; DODDS, KG ; EDE, AJ ; PIERSON, CA ;
MONTGOMERY, GW ; GARMONSWAY, HG ; BEATTIE, AE ; DAVIES, K ;
MADDOX, JF ; KAPPES, SM ; STONE, RT ; NGUYEN, TC ; PENTY, JM
; LORD, EA ; BROOM, JE ; BUITKAMP, J ; SCHWAIGER, W ;
EPPLEN, JT ; MATTHEW, P ; MATTHEWS, ME ; HULME, DJ ; BEH,
DJ ; MCGRAW, RA ; BEATTIE, CW ;

Cattle Marker **BM2113** (36)
Chromosome: 02 Locus: BM2113
Relative Position: 106.2 cM
Region: Not physically assigned
Database Name: GENBANK Accession Number: M97162
Heterozygosity: 72%; Informative Meioses: 310; Number of Al-
leles: 11
Minimum Allele Size: 123 (bp)
Maximum Allele Size: 143 (bp)
Annealing Temperature: 58 °C
Forward Primer Sequence: GCTGCCTTCTACCAAATACCC
Reverse Primer Sequence: CTTCCTGAGAGAAGCAACACC

References:

Title: "A GENETIC LINKAGE MAP FOR CATTLE"
Journal: GENETICS, 1994, Volume: 136, Page: 619
Authors: BISHOP, MD ; KAPPES, SM ; KEELE, JW ; STONE, RT ;
SUNDEN, SLF; HAWKINS, GA ; SOLINAS TOLDO, S ; FRIES, R ;
GROSZ, MD ; YOO, J ; BEATTIE, CW ;

Title: "A SECOND-GENERATION LINKAGE MAP OF THE BOVINE GENOME"
Journal: GENOME RES, 1997, Volume: 7, Page: 235
Authors: KAPPES, SM ; KEELE, JW ; STONE, RT ; MCGRAW, RA ;
SONSTEGARD, TS ; SMITH, TPL; LOPEZ-CORRALES, NL ; BEATTIE, CW
;

Cattle Marker **BMS522** (1183)

Chromosome: 07 Locus: BMS522
Relative Position: 120.7 cM
Region: Not physically assigned
Heterozygosity: 41%; Informative Meioses: 101; Number of Al-
leles: 5
Minimum Allele Size: 146 (bp)
Maximum Allele Size: 154 (bp)
Annealing Temperature: 58 °C
Forward Primer Sequence: CTTGCTTACTGCTTGCTATGAA
Reverse Primer Sequence: CCCAACAAAATTTCTGATTCTC

References:

Title: "A SMALL-INSERT BOVINE GENOMIC LIBRARY HIGHLY ENRICHED
FOR MICROSATELLITE REPEAT SEQUENCES"
Journal: MAMM GENOME, 1995, Volume: 6, Page: 714
Authors: STONE, RT ; PULIDO, JC ; DUYK, GM ; KAPPES, SM ;
KEELE, JW ; BEATTIE, CW ;

Title: "A SECOND-GENERATION LINKAGE MAP OF THE BOVINE GENOME"
Journal: GENOME RES, 1997, Volume: 7, Page: 235
Authors: KAPPES, SM ; KEELE, JW ; STONE, RT ; MCGRAW, RA ;
SONSTEGARD, TS ; SMITH, TPL; LOPEZ-CORRALES, NL ; BEATTIE, CW
;

Cattle Marker BMC2228 (731)
Chromosome: 29 Locus: BMC2228
Relative Position: 0 cM
Region: Not physically assigned
Database Name: GENBANK Number: G19101
Heterozygosity: 48%; Informative Meioses: 194; Number of Al-
leles: 3
Minimum Allele Size: 171 (bp)
Maximum Allele Size: 177 (bp)
Annealing Temperature: 58 °C
Forward Primer Sequence: GGCATGGCATAACCATATAGC
Reverse Primer Sequence: ATGGTGGTGTCAGATTGGC

References:

Title: "A SECOND-GENERATION LINKAGE MAP OF THE BOVINE GENOME"
Journal: GENOME RES, 1997, Volume: 7, Page: 235
Authors: KAPPES, SM ; KEELE, JW ; STONE, RT ; MCGRAW, RA ;
SONSTEGARD, TS ; SMITH, TPL; LOPEZ-CORRALES, NL ; BEATTIE, CW
;

Title: "CHARACTERIZATION OF ELEVEN BOVINE MICROSATELLITES FROM
COSMID CLONES"
Journal: ANIM GENET, 1997, Volume: 28, Page: 238
Authors: KAPPES, SM ; HAWKINS, GA ; BISHOP, MD ; BEATTIE, CW
;

Cattle Marker BM1824 (14)
Chromosome: 01 Locus: BM1824
Relative Position: 108.6 cM
Region: Not physically assigned
Database Name: GENBANK Accession Number: G18394
Heterozygosity: 62%; Informative Meioses: 319; Number of Al-
leles: 7
Minimum Allele Size: 178 (bp)
Maximum Allele Size: 192 (bp)
Annealing Temperature: 58 °C
Forward Primer Sequence: GAGCAAGGTGTTTTTCCAATC
Reverse Primer Sequence: CATTCTCCAACTGCTTCCTTG

References:

Title: "A GENETIC LINKAGE MAP FOR CATTLE"
Journal: GENETICS, 1994, Volume: 136, Page: 619
Authors: BISHOP, MD ; KAPPES, SM ; KEELE, JW ; STONE, RT ;
SUNDEN, SLF; HAWKINS, GA ; SOLINAS TOLDO, S ; FRIES, R ;
GROSZ, MD ; YOO, J ; BEATTIE, CW ;

Title: "A SECOND-GENERATION LINKAGE MAP OF THE BOVINE GENOME"
Journal: GENOME RES, 1997, Volume: 7, Page: 235
Authors: KAPPES, SM ; KEELE, JW ; STONE, RT ; MCGRAW, RA ;
SONSTEGARD, TS ; SMITH, TPL; LOPEZ-CORRALES, NL ; BEATTIE, CW
;

Cattle Marker BM875 (162)
Chromosome: 10 Locus: BM875
Relative Position: 46.5 cM
Region: Not physically assigned
Database Name: GENBANK Accession Number: G18482
Heterozygosity: 62%; Informative Meioses: 229; Number of Al-
leles: 7
Minimum Allele Size: 93 (bp)
Maximum Allele Size: 111 (bp)
Annealing Temperature: 58 °C
Forward Primer Sequence: ACCTATCTCATTTGGCTTCTGG
Reverse Primer Sequence: AAAAAACCCCAAACAACAACC

References:

Title: "A GENETIC LINKAGE MAP FOR CATTLE"
Journal: GENETICS, 1994, Volume: 136, Page: 619
Authors: BISHOP, MD ; KAPPES, SM ; KEELE, JW ; STONE, RT ;
SUNDEN, SLF; HAWKINS, GA ; SOLINAS TOLDO, S ; FRIES, R ;
GROSZ, MD ; YOO, J ; BEATTIE, CW ;

Title: "A SECOND-GENERATION LINKAGE MAP OF THE BOVINE GENOME"
Journal: GENOME RES, 1997, Volume: 7, Page: 235

Authors: KAPPES, SM ; KEELE, JW ; STONE, RT ; MCGRAW, RA ;
SONSTEGARD, TS ; SMITH, TPL; LOPEZ-CORRALES, NL ; BEATTIE,
CW;

Cattle Marker TGLA73 (673)
Chromosome: 09 Locus: TGLA73
Relative Position: 71.4 cM
Region: Not physically assigned
Heterozygosity: 48%; Informative Meioses: 229; Number of Al-
leles: 7
Minimum Allele Size: 116 (bp)
Maximum Allele Size: 130 (bp)
Annealing Temperature: 58 °C
Forward Primer Sequence: GAGAATCACCTAGAGAGGCA
Reverse Primer Sequence: CTTTCTCTTTAAATTCTATATGGT

References:

Title: "POLYMORPHIC DNA MARKERS IN BOVIDAE (WORLD INTELLECTUAL
PROPERTY ORG GENEVA)"
Journal: WO PUBL NO 92/13102, 1992, Volume: , Page:
Authors: GEORGES, M ; MASSEY, J ;

Title: "AN AUTOSOMAL GENETIC LINKAGE MAP OF THE SHEEP GENOME"
Journal: GENETICS, 1995, Volume: 140, Page: 703
Authors: CRAWFORD, AM ; DODDS, KG ; EDE, AJ ; PIERSON, CA ;
MONTGOMERY, GW ; GARMONSWAY, HG ; BEATTIE, AE ; DAVIES, K ;
MADDOX, JF ; KAPPES, SM ; STONE, RT ; NGUYEN, TC ; PENTY, JM
; LORD, EA ; BROOM, JE ; BUITKAMP, J ; SCHWAIGER, W ;
EPPLEN, JT ; MATTHEW, P ; MATTHEWS, ME ; HULME, DJ ; BEH,
DJ ; MCGRAW, RA ; BEATTIE, CW ;

Title: "A SECOND-GENERATION LINKAGE MAP OF THE BOVINE GENOME"
Journal: GENOME RES, 1997, Volume: 7, Page: 235
Authors: KAPPES, SM ; KEELE, JW ; STONE, RT ; MCGRAW, RA ;
SONSTEGARD, TS ; SMITH, TPL; LOPEZ-CORRALES, NL ; BEATTIE, CW
;

Cattle Marker BM1818 (11)
Chromosome: 23 Locus: BM1818
Relative Position: 50.9 cM
Region: Not physically assigned
Database Name: GENBANK Accession Number: G18391
Heterozygosity: 90%; Informative Meioses: 330; Number of Al-
leles: 8
Minimum Allele Size: 258 (bp)
Maximum Allele Size: 272 (bp)
Annealing Temperature: 56 °C
Forward Primer Sequence: AGCTGGGAATATAACCAAAGG
Reverse Primer Sequence: AGTGCTTTCAAGGTCCATGC

References:

Title: "A GENETIC LINKAGE MAP FOR CATTLE"
Journal: GENETICS, 1994, Volume: 136, Page: 619
Authors: BISHOP, MD ; KAPPES, SM ; KEELE, JW ; STONE, RT ;
SUNDEN, SLF; HAWKINS, GA ; SOLINAS TOLDO, S ; FRIES, R ;
GROSZ, MD ; YOO, J ; BEATTIE, CW ;

Title: "A SECOND-GENERATION LINKAGE MAP OF THE BOVINE GENOME"
Journal: GENOME RES, 1997, Volume: 7, Page: 235
Authors: KAPPES, SM ; KEELE, JW ; STONE, RT ; MCGRAW, RA ;
SONSTEGARD, TS ; SMITH, TPL; LOPEZ-CORRALES, NL ; BEATTIE, CW
;

Appendix 3: Amplified RFLP with MSHR P9 / P12 and MspI restric-
tion.

BT39469 standard; DNA; MAM; 954 BP.
U39469;
U39469.2
26-NOV-1995 (Rel. 45, Created)
22-OCT-1999 (Rel. 61, Last updated, Version 3)
Bos taurus alpha melanocyte stimulating hormone receptor
(MSHR)gene, complete cds.
Bos taurus
Eukaryota; Metazoa; Chordata; Craniata; Vertebrata; Teleostomi;
Euteleostomi; Mammalia; Eutheria; Cetartiodactyla; Ruminantia;
Pecora; OC Bovoidea; Bovidae; Bovinae; Bos.
[1]
1-954
MEDLINE; 96325106.
Joerg H., Fries H.R., Meijerink E., Stranzinger G.F.;
"Red coat color in Holstein cattle is associated with a deletion
in the MSHR gene";
Mamm. Genome 7(4):317-318(1996).
[2]
1-954
Joerg H., Fries H.R., Meijerink E., Stranzinger G.F.;
Submitted (26-OCT-1995) to the EMBL/GenBank/DDBJ databases.
Hannes Joerg, Gruppe Zuchtungsbiologie, Institut fur
Nutztierwissenschaften, Tannenstr.1, Zurich, CH CH-8092, Swit-
zerland
[3]
Sequence update by submitter
1-954
Joerg H., Fries H.R., Meijerink E., Stranzinger G.F.;
Submitted (21-OCT-1999) to the EMBL/GenBank/DDBJ databases.
Hannes Joerg, Gruppe Zuchtungsbiologie, Institut fur
Nutztierwissenschaften, Tannenstr.1, Zurich, CH CH-8092, Swit-
zerland
SPTREMBL; Q28025; Q28025.

On Oct 21, 1999 this sequence version replaced gi:1073240.
Key                 Location/Qualifiers
source              1. .954
/db_xref="taxon:9913"
/organism="Bos taurus"
mRNA                <1. .>954
/gene="MSHR"
/product="alpha melanocyte stimulating hormone receptor"
CDS                 1. .954
/codon_start=1
/db_xref="SPTREMBL:Q28025"
/gene="MSHR"
/product="alpha melanocyte stimulating hormone receptor"
/protein_id="AAC48590.2"
/translation="MPALGSQRRLLGSLNCTPPATLPFTLAPNRTGPQCLEVSIPDGLFLSLGL
VSLVENVLVVAAIAKNRNLHSPMYYFICCLAVSDLLVSVSNVLETAVMPLLEAGVLATQAAVVQ
QLDNVIDVLICGSMVSSLCFLGAIAVDRYISIFYALRYHSVVTLPRAWRIIAAIWVASILTSLL
FITYYNHKVILLCLVGLFIAMLALMAVLYVHMLARACQHARGIARLQKRQRPIHQGFGLKGAAT
LTILLGVFFLCWGPFFLHLSLIVLCPQHPTCGCIFKNFNLFLALIICNAIVDPLIYAFRSQELR
KTLQEVLQCSW"

Sequence 954 BP; 142 A; 343 C; 251 G; 218 T; 0 other;

BT39469  Length: 954  May 16, 2000 14:01  Type: N  Check: 6206
..

  1  ATGCCTGCAC TTGGCTCCCA GAGGCGGCTG CTGGGTTCCC TTAACTGCAC

 51  GCCCCCAGCC ACCCTCCCCT TCACCCTGGC CCCCAACCGG ACGGGGCCCC

101  AGTGCCT[?][?][?] [?][?][?][?][?][?][?][?][?][?] [?][?][?][?][?][?][?][?]GC TCTTTCTCAG CCTGGGGCTG

151  GTGAGTCTCG TGGAGAACGT GCTGGTAGTG GCTGCCATTG CCAAGAACCG

201  CAACCTGCAC TCCCCCATGT ACTACTTTAT CTGCTGCCTG GCTGTGTCTG

251  ACTTGCTGGT GAGCGTCAGC AACGTGCTGG AGACGGCAGT CATGCCGCTG

301  CTGGAGG[?][?][?] TGTCCTGGC CACCCAGGCG GCCGTGGTGC AGCAGCTGGA

351  CAATGTCATC GACGTGCTCA TCTGCGGATC CATGGTGTCC AGCCTCTGCT

401  TCCTGGGTGC CATTGCTGTG GACCGCTACA TCTCCATCTT CTACGCCCTG

451  CGGTACCACA GTGTTGTGAC ACTGCCCCGA GCGTGGAGGA TCATTGCGGC

501  CATCTGGGTG GCCAGCATCC TCACCAGCCT GCTCTTCATC ACCTACTACA

551  ACCACAAGGT CATCCTGCTG TGCCTCGTTG GCCTCTTCAT AGCTATGCTG

601  GCCCTGATGG CCGTCCTCTA CGTCCACATG CTGGCCCGGG CCTGCCAGCA

651  TGCCCGGGGC ATTGCCCGGC TCCAGAAGAG GCAGCGCCCC ATTCATCAGG

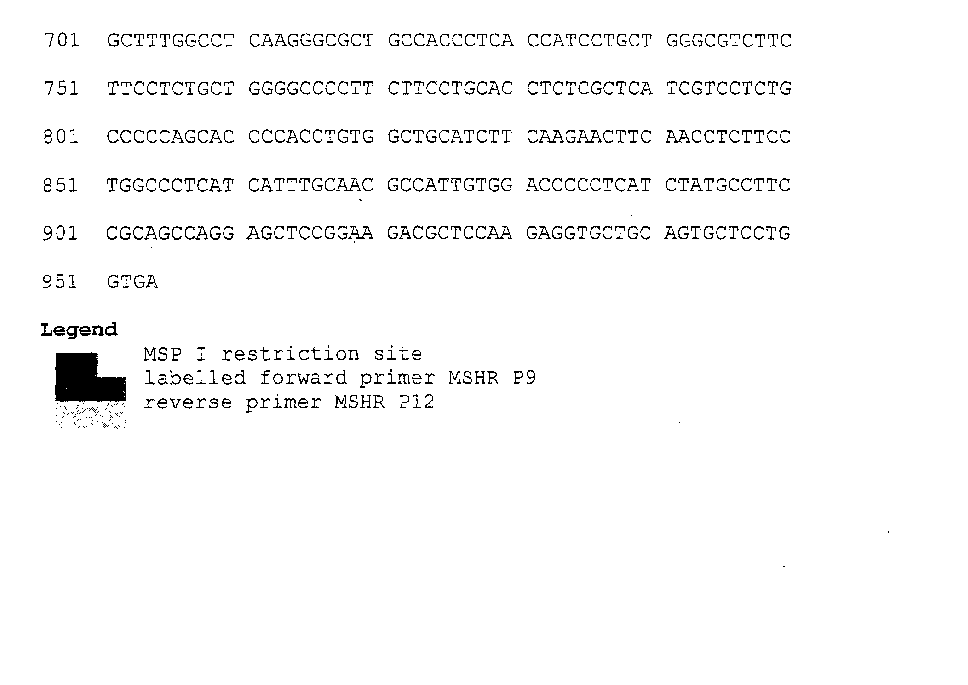
701 GCTTTGGCCT CAAGGGCGCT GCCACCCTCA CCATCCTGCT GGGCGTCTTC

751 TTCCTCTGCT GGGGCCCCTT CTTCCTGCAC CTCTCGCTCA TCGTCCTCTG

801 CCCCCAGCAC CCCACCTGTG GCTGCATCTT CAAGAACTTC AACCTCTTCC

851 TGGCCCTCAT CATTTGCAAC GCCATTGTGG ACCCCCTCAT CTATGCCTTC

901 CGCAGCCAGG AGCTCCGGAA GACGCTCCAA GAGGTGCTGC AGTGCTCCTG

951 GTGA

Legend
 MSP I restriction site
labelled forward primer MSHR P9
reverse primer MSHR P12

## Claims

1. A method for determining genetic traits of improved breed animal embryos prior to their implantation into a female wherein single nucleotide polymorphisms and/or polymorphic microsatellite markers are identified simultaneously.

2. The method according to claim 1 wherein the improved breed animal embryo is a bovine embryo.

3. The method according to claim 1 or 2, wherein the sex of an improved breed embryo is determined by the size of PCR products whereby the presence of two PCR products of the same genetic locus indicates a female embryo and the presence of one PCR product indicates a male embryo.

4. The method according to claim 3 wherein the genetic trait to be determined is the sex of an improved breed animal embryo by identifying microsatellite markers on the X chromosome, said method comprising the following steps:
a) isolation of an embryo cell or cells at the blastula stage by microsection or biopsy;
b) denaturating DNA from said cells by alternately freezing and thawing the cells;
c) performing polymerase chain reaction (PCR) on said denatured DNA to amplify a fragment homologous to a region of the X chromosome comprising microsatellite markers; and
d) evaluating the size of the fragments obtained from step c.

5. The method according to claim 1 or 2 wherein the genetic traits to be determined are the coat colour and the sex, said method comprising the following steps:
a) isolation of an embryo cell or cells at the blastula stage by microsection or biopsy;
b) denaturating DNA from said cells by alternately freezing and thawing the cells;
c) performing a first round of polymerase chain reaction (PCR) on said denatured DNA to amplify a fragment homologous to a region of the X chromosome comprising microsatellite markers and single nucleotide polymorphisms using specific primers;
d) performing a second round of PCR on a DNA aliquot from the first PCR round using primers of microsatellite markers in one reaction and the primers of a melanocyte stimulating hormone receptor in another reaction.
e) performing a restriction enzyme digest with the DNA derived from PCR with the two primers of the melanocyte stimulating hormone receptor from step d); and
f) evaluating the size of the fragments obtained from step d and step e.

6. The method according to claim 5, wherein the primers from steps d) and e) are derived from the bovine melanocyte stimulating hormone receptor GenBank accession number BT39469 (http://www.ncbi.nlm.nih.gov:80/entrez/query.fcgi?cmd=Retrieve&d b=Nucleotide&list_uids=6091552&dopt=GenBank).

7. The method according to claim 1 or 2 wherein the parentage of an improved breed animal embryo is determined by identifying microsatellite markers, said method comprising the following steps:
a) isolation of an embryo cell or cells at the blastula stage by microsection or biopsy;
b) denaturating DNA from said cells by alternately freezing and thawing the cells;
c) performing of polymerase chain reaction (PCR) amplification on said denatured DNA to amplify fragments of different chromosomes comprising microsatellite markers; and
d) evaluating the size of the fragments obtained from step c.

8. The method according to anyone of claims 1-3, wherein the sex of an improved breed animal embryo is determined by identifying single nucleotide polymorphism on the X chromosome, said method comprising the following steps:
a) isolation of an embryo cell or cells at the blastula stage by microsection or biopsy;
b) denaturating DNA from said cells by alternately freezing and thawing the cells;
c) performing polymerase chain reaction (PCR) on said denatured DNA to amplify a fragment homologous to a region of the X chromosome comprising single nucleotide polymorphisms; and
d) evaluating the sequence of the fragments obtained from step c.

9. The method according to claim 8 wherein the parentage of an improved breed animal embryo is determined by identifying single nucleotide polymorphisms.

10. A laboratory kit for determining simultaneously several genetic traits like sex, parentage and quantitative trait loci of an improved breed animal embryo, comprising oligonucleotide primers selected from autosomal and sex chromosome specific markers.

11. The laboratory kit according to claim 10, wherein the oligonucleotide primers are selected from the groups consisting of:
a) Cattle Marker BMS631 Forward Primer Sequence: TTTCCACCCTCAGCTAAAGTG Reverse Primer Sequence: AAATGACAGGTGTCTTGGGC, Cattle Marker BMS1616 Forward Primer Sequence: CAGTGTGTATAGCATGATTCCG Reverse Primer Sequence: AGTTGGTCTGCATTCATACATTAA, and
Cattle Marker BMC6021 Forward Primer Sequence: AGGCCAAGTGAAGAGGTCAC Reverse Primer Sequence: TCTGCTCCAGCTTTCCAGTA for sexing;
b) Cattle Marker TGLA 227 Forward Primer Sequence: CGAATTCCAAATCTGTTAATTTGCT Reverse Primer Sequence: ACAGACAGAAACTCAATGAAAGCA Cattle Marker BM6465 Forward Primer Sequence: AGGAGCAAGCATCTTTAATGTG Reverse Primer Sequence: TGCCAGGCTATAGAAGGACTT Cattle Marker TGLA122 Forward Primer Sequence: CCCTCCTCCAGGTAAATCAGC Reverse Primer Sequence: AATCACATGGCAAATAAGTACATAC Cattle Marker BMS779 Forward Primer Sequence: TCTTGTGGGGAACCTCTA Reverse Primer Sequence: TGCTTGGAAGTATACATGATGG Cattle Marker AGLA293 Forward Primer Sequence: GAAACTCAACCCAAGACAACTCAAG Reverse Primer Sequence: ATGACTTTATTCTCCACCTAGCAGA Cattle Marker BM2113 Forward Primer Sequence: GCTGCCTTCTACCAAATACCC Reverse Primer Sequence: CTTCCTGAGAGAAGCAACACC Cattle Marker BMS522 Forward Primer Sequence: CTTGCTTACTGCTTGCTATGAA Reverse Primer Sequence: CCCAACAAAATTTCTGATTCTC Cattle Marker BMC2228 Forward Primer Sequence: GGCATGGCATAACCATATAGC Reverse Primer Sequence: ATGGTGGTGTCAGATTGGC Cattle Marker BM1824 Forward Primer Sequence: GAGCAAGGTGTTTTTCCAATC Reverse Primer Sequence: CATTCTCCAACTGCTTCCTTG Cattle Marker BM875 Forward Primer Sequence: ACCTATCTCATTTGGCTTCTGG Reverse Primer Sequence: AAAAAACCCCAAACAACAACC Cattle Marker TGLA73 Forward Primer Sequence: GAGAATCACCTAGAGAGGCA Reverse Primer Sequence: CTTTCTCTTTAAATTCTATATGGT Cattle Marker BM1818 Forward Primer Sequence: AGCTGGGAATATAACCAAAGG Reverse Primer Sequence: AGTGCTTTCAAGGTCCATGC for parentage control and
c) Cattle MSHR Forward Primer Sequence: GGAGGTGTCCATCCCTGACGG Reverse Primer Sequence: CTGCTGCACCACGGCCGCCTG For determining coat colour.
